# EUROPEAN PATENT APPLICATION

(11) **EP 4 420 664 A1**
(43) Date of publication of application: **28.08.2024**
(21) Application number: 23158767.6
(22) Date of filing: 27.02.2023
(51) Int. Cl.: A61K 31/439, A61P 25/34, A61K 9/08, A61K 47/02

(54) **PHARMACEUTICAL COMPOSITION COMPRISING CYTISINE**

(71) Applicant: Adamed Pharma S.A., 05-152 Czosnow (PL)
(72) Inventor: LYSIAK, Magdalena, 01-991 Warszawa (PL); ZARZYCKA, Mariola, 01-915 Warszawa (PL); RZASA, Joanna, 35-116 Rzeszow (PL)
(74) Representative: Sulikowski, Daniel

(57) **Abstract**

The present invention relates to a stabilized composition with significantly improved shelf-life stability. More particularly, the present invention relates to a stable liquid aqueous pharmaceutical composition comprising cytisine or a pharmaceutically acceptable salt thereof, at least one antioxidant selected from the group consisting of a sulfite, bisulfite, and metabisulfite compound. The present invention relates also to a use of at least one antioxidant selected from the group consisting of a sulfite, bisulfite, and metabisulfite compound for the stabilization of a liquid aqueous pharmaceutical composition comprising cytisine.

## Description

### Technical Field

The present invention relates to a stabilized composition with significantly improved shelf-life stability. More particularly, the present invention relates to a stabilized liquid aqueous pharmaceutical composition comprising cytisine or a pharmaceutically acceptable salt thereof and at least one antioxidant selected from the group consisting of a sulfite, bisulfite, and metabisulfite compound.

### State of the art

Tobacco smoking is the most widespread and serious addiction and a major cause of death from cancer and cardiovascular and pulmonary diseases, with more than 8 million people dying from a tobacco-related disease per year worldwide. Smoking cessation reduces the risk of premature death associated with continued smoking by about 90%. However, smoking cessation is extremely difficult for several reasons, among which includes the complexity of nicotine addiction.

Nicotine is the main psychoactive component of tobacco responsible for addiction. Nicotine addiction is associated with repeated nicotine ingestion from cigarettes leading to stimulation of brain nAChRs and release of dopamine and other neurotransmitters from nucleus accumbens, ventral tegmental area (VTA) and frontal cortex area.

In the United States, the current Food and Drug Administration-approved smoking cessation medications are varenicline and bupropion. Similar to varenicline, its synthetic derivative, cytisine, acts as a selective partial agonist at α4β2α4β2 and binds to nicotinic acetylcholine receptors with an affinity that is higher than that of nicotine.

Cytisine, also known as baptitoxine, cytisinicline, or sophorine is the oldest medication licensed and used in central and eastern Europe for smoking cessation, and products containing cytisine are available in central and eastern European countries, such as Russia and Poland, western Asia, and Canada.

Cytisine is a plant-based alkaloid derived primarily from Leguminosae plants (especially the seeds of Laburnum anagyroides). It is a nicotinic acetylcholine receptor (nAChR) partial agonist that has been used successfully in smoking cessation therapy.

IUPAC name of cytisine is (1R,5S)- 1,2,3,4,5,6- hexahydro- 1,5-methano-8H-pyrido[1,2a] [1,5] diazocin-8-one and has the following structure of formula (I):

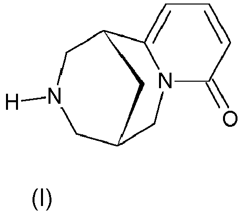

Cytisine is currently available in tablet (Tabex^{®}, Recigar^{®}) and capsule (Desmoxan) forms, with a complex dosing regimen that requires one cytisine dose (tablet or capsule: 1.5 mg) every 2 hours at first (6 doses per day), then titrated downward over 25 days to 2 doses per day.

The complex dosage regimen necessitates the development of a more convenient method for cytisine oral administration, particularly in the form of a liquid, mist, spray, or aerosol.

The international patent application WO2014201735 discloses an oral nicotine-substituted cytisine atomized liquid composition containing the following components in mass percentage per 1 L of the oral atomized liquid: 0.1-10% tobacco, 0.3-15% cocoa extract, 0.1-0.9% cytisine, 0.1-0.5% TWEEN 80, and 75-90% primer.

The patent RU2593585C1 discloses an intranasal solution containing cytisine, water, sodium hydrogen phosphate and sodium dihydrogen phosphate as acidity regulators, EDTA, methylparaben (Paraben M) and propylparaben (Paraben P) as preservatives, sodium chloride as salt, polysorbate as co-solvent, and citric acid as antioxidant.

The international patent application WO2021115977A1 discloses a liquid pharmaceutical composition containing cytisine, water and one or more pharmaceutical excipients, including at least one inorganic pH regulating agent chosen from the group consisting of inorganic acids, inorganic buffers, and inorganic acid salts, with a pH value ranging from 3.0 to 7.5.

The patent application EP3967298A1 discloses an aerosol composition comprising cytisine, a hydrophilic non-ionic emulsifier from the group of copolymers or polymers, at least one alcohol, water and one or more excipients, wherein the composition has a pH of 7-10.

The polish patent application PL416496 discloses a solution for inhalation and/or nebulization with atomizers and/or vaporizers used in e-cigarettes. The solution contains a dose of 0.66% of cytisine and/or 0.02% of nicotine to maximum limit values necessary to maintain a liquid unsaturated solution in thermodynamic room temperature thermal comfort at hygroscopic equilibrium, with the maximum concentration of cytisine and/or nicotine for these conditions not exceeding 99.99%.

Unfortunately, the inventor(s) of the present invention found that the liquid composition containing cytisine and intended to be administered as a liquid, mist, spray, or aerosol has some disadvantages, most notably, the presence of N-nitroso cytisine impurity above its acceptable intake (Al) limit in the liquid composition which most likely formed during the formulation stage or during it shelf-life, which was not previously reported in the prior art.

Due to elevated levels of nitrosamine impurities, an increasing number of drug products have been suspended or recalled from the market. Some nitrosamines are classified as probable or possible carcinogens in humans. One of these incidents involved the smoking cessation medication varenicline, which Pfizer recalled from the US market in 2021 because it might have contained levels of the N-nitroso-varenicline impurity that were at or above those allowed by the US Food and Drug Administration.

The presence of nitrosamine impurities in marketed drug products is becoming a significant focus for the pharmaceutical industry following regulatory guidelines on identification, permitted limits and mitigation strategy to reduce below acceptable levels. There are several ways in which nitrosamine impurities can form. According to published CHMP Article 5(3) opinion and the Q&A document on its implementation EMA/409815/2020, there are several underlying factors that contribute to the formation of N-nitrosamines in pharmaceutical products. Some of these factors include:
- The reaction of nitrosatable nitrogen functionality in APIs or their impurities / degradants with nitrosating agents present in finished product components during formulation or storage. Several examples have been reported where the amine functionality was shown to be vulnerable to nitrosation and formation of the corresponding N-nitroso impurity (i.e., NO-API). Secondary amines appear particularly vulnerable to this reaction although some cases with tertiary amines have also been observed. Vulnerable amines could also be formed by degradation (e.g., hydrolysis) during formulation or storage.
- Oxidation of hydrazine or other amine-containing functional groups present in active substances or their impurities/degradants (e.g., from hydrazones and hydrazides), either in active substance manufacturing processes or during storage. This root cause has also been observed during manufacture and storage of finished products containing such functional groups. Potential oxidants include oxygen and peroxides (common impurities in some excipients).

The present inventor(s) found that cytisine degrades during the formulation of a liquid aqueous formulation because it contains an aliphatic secondary amine group that degrades through a variety of processes, including oxidative degradation and/or interactions with other ingredients, including but not limited to reactions with nitrite impurities in excipients, which form nitrosating agents under specific conditions and can react with vulnerable secondary amines to form N-Nitroso cytisine impurity above its acceptable intake limit.

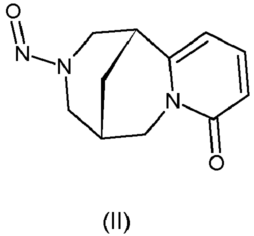

Therefore, it is necessary to develop stable pharmaceutical compositions containing cytisine or a pharmaceutically acceptable salt thereof, with an improved shelf-life and a significantly reduced susceptibility to oxidative degradation and other forms of degradation such as nitrosation during the shelf- life.

### Summary of the invention

The inventor(s) of the present invention found that an improved stability, in particularly stability against nitrosation and/or oxidative degradation as well as chemical stability, can be obtained by using at least one antioxidant selected from the group consisting of a sulfite, bisulfite, and metabisulfite compound in the composition, wherein at least one antioxidant is present in a specific range, based on the total weight of the pharmaceutical composition in a specific pH range.

The object of the present invention is a liquid aqueous pharmaceutical composition comprising cytisine or a pharmaceutically acceptable salt thereof, at least one antioxidant selected from the group consisting of a sulfite, bisulfite, and metabisulfite compound, wherein at least one antioxidant is present in an amount of from 0.01 % to 2.0 % by weight, based on the total weight of the pharmaceutical composition, and wherein the pH of the said liquid aqueous composition is in the range of 5.0-7.0.

Another aspect of the present invention is the use of said liquid aqueous pharmaceutical composition for use in the treatment of tobacco smoking addiction and other forms of nicotine addiction.

Another aspect of the present invention is the use of at least one antioxidant selected from the group consisting of a sulfite, bisulfite, and metabisulfite compound for the stabilization of a liquid aqueous pharmaceutical composition containing cytisine.

### Definitions

Along the present description as well as in the claims, the singular expressions, generally preceded by the articles "a", "an" or "the", are meant to also include the plural forms, unless the context clearly indicates otherwise. Numeric ranges defined by lower and upper endpoints are meant to include also said stated endpoints. The percentage disclosed (%) are always weight percentage, based on the total weight of the pharmaceutical composition unless clearly stated otherwise.

The term "antioxidant" refers to a substance capable of slowing or preventing the oxidation of other substances. Oxidation reactions can generate free radicals, which can set off chain reactions that can be damaging. Antioxidants prevent these chain reactions by removing free radical intermediates and inhibit other oxidation reactions by being oxidized themselves; thus, antioxidants can be considered reducing agents.

The term "nitrosation" refers to reactions in which the NO group is introduced into an organic molecule.

The term "a sulfite" refers to any salt comprising the anion SO₃²⁻.

The term "a bisulfite" refers to any salt comprising the anion HSO₃⁻.

The term "a metabisulfite" refers to any salt comprising the anion S₂O₅²⁻.

The term "acceptable intake (Al) limit" refers to the upper limit of N-Nitrosamines that can be present in the medicinal product and are classified as "cohort of concern" substances according to ICH M7(R1) guidelines, and in the context of the present invention, it refers to the upper limit of N-nitroso cytisine impurity that can be present in the liquid aqueous pharmaceutical composition containing cytisine.

The term "stabilization" in the context of the present invention refers to the process of protecting a liquid aqueous composition from nitrosation and/or oxidative degradation, as well as any thermal or chemical degradation of the liquid aqueous composition.

### Detailed description of the invention

The first aspect of the present invention is a liquid aqueous pharmaceutical composition comprising cytisine or a pharmaceutically acceptable salt thereof, at least one antioxidant selected from the group consisting of a sulfite, bisulfite, and metabisulfite compound, wherein at least one antioxidant is present in an amount of from 0.01 % to 2.0 % by weight, based on the total weight of the pharmaceutical composition and wherein the pH of the said liquid aqueous composition is in the range of 5.0-7.0.

In comparison to prior art compositions, the present invention offers improved stability, particularly stability against nitrosation and/or oxidative degradation as well as chemical stability and thus enhances the shelf-life of the pharmaceutical composition using at least one antioxidant selected from the group consisting of a sulfite, bisulfite, and metabisulfite compound.

In one embodiment of the present invention, cytisine or a pharmaceutically acceptable salt thereof is present in an amount which corresponds to from 0.1 % to 10 % by weight of cytisine free base, based on the total weight of the pharmaceutical composition.

In one embodiment, a pharmaceutically acceptable salt of cytisine may include, but is not limited to, an addition salt of inorganic or organic acids, such as hydrochloric acid, hydrobromic acid, phosphoric acid, sulphuric acid, tartaric acid, lactic acid, succinic acid, and formic acid.

The terms sulfite, bisulfite and metabisulfite will be understood to mean sulfite ion (SO₃²⁻) bisulfite ion (HSO₃⁻), and metabisulfite ion (S₂O₅²⁻) respectively, derivable from any pharmaceutically acceptable source or precursor (that is, sulfite, bisulfite and metabisulfite compound), such source or precursor being illustrated, without limitation, by ammonium, alkalimetal, alkaline earth metal, and amine salts and mixed salts of an alkali metal and an organic compound. Alkaline metal salts include sodium and potassium salts, alkaline earth metal salts include calcium, magnesium, strontium, and barium salts, and amine salts are salts of an amine that is a primary, secondary, or tertiary lower-alkylamine such as methylamine, ethylamine, isopropyl amine, n-butylamine, diethyl amine, triethylamine, and others.

In one embodiment of the present invention, at least one antioxidant is selected from sodium sulfite, potassium sulfite, sodium bisulfite, potassium bisulfite, sodium metabisulfite and potassium metabisulfite.

In the preferred embodiment, at least one antioxidant is sodium sulfite, sodium bisulfite and/or sodium metabisulfite.

In one embodiment the liquid aqueous pharmaceutical composition of the present invention, at least one antioxidant is present in an amount of from 0.01% to 2.0% by weight, more preferably is comprised between 0.01 % and 1.0% by weight, in each case based on the total weight of the pharmaceutical composition.

In the most preferred variant of the above embodiment, at least one antioxidant is sodium sulfite, sodium bisulfite or sodium metabisulfite and is present in an amount of from 0.01% to 2.0% by weight, more preferably is comprised between 0.01% and 1.0% by weight, in each case based on the total weight of the pharmaceutical composition.

In one embodiment of the present invention, the pH of the liquid aqueous pharmaceutical composition is in the range of 5.0-7.0.

In one embodiment of the present invention, the pH of liquid aqueous composition is preferably in the range of 5.2-6.7, more preferably 5.5-6.4, and most preferably 5.8-6.1.

In the most preferred variant of the present invention, the pH of a liquid aqueous pharmaceutical composition is adjusted using an inorganic pH-regulating agent.

In one embodiment of the present invention, the inorganic pH-regulating agent is at least one inorganic salt, wherein an inorganic salt is a salt obtained by a partial neutralization of inorganic diprotic or polyprotic acids. Preferably, the inorganic diprotic or polyprotic acids are sulfuric acid and phosphoric acid. More preferably, the inorganic salt is selected from disodium hydrogen phosphate, sodium dihydrogen phosphate dihydrate, dipotassium hydrogen phosphate and potassium dihydrogen phosphate or mixture thereof.

In one embodiment of the present invention, the inorganic pH regulating agent is at least one inorganic salt, wherein inorganic salt is selected from disodium hydrogen phosphate, sodium dihydrogen phosphate dihydrate, dipotassium hydrogen phosphate and potassium dihydrogen phosphate or mixture thereof.

In one embodiment of the present invention, the inorganic pH regulating agent is at least one inorganic salt, wherein inorganic salt is selected from disodium hydrogen phosphate, sodium dihydrogen phosphate dihydrate, dipotassium hydrogen phosphate and potassium dihydrogen phosphate or mixture thereof and is present in an amount from 0.05% to 10% by weight, based on the total weight of the pharmaceutical composition.

In the most preferred embodiment, the composition of the present invention does not comprise any further organic pH-regulating agents.

Cytisine has an aliphatic secondary amine group and is susceptible to degradation during the manufacturing process or storage by nitrosation and/or oxidative degradation reactions to form the corresponding N-nitrosamine impurity, i.e., N-nitroso cytisine. Nitrosamine impurities are potentially carcinogenic, and all MAHs/Applicants of human medicinal products should ensure that the presence of nitrosamine impurities in their medicinal products is mitigated as much as possible and controlled at or below a limit defined based on ICH M7(R1) principles for "cohort of concern" substances in the medicinal product.

The assessment report of the CHMP's opinion on nitrosamine impurities in human medicinal products pursuant to Article 5(3) of Regulation (EC) No 726/2004 offers general guidelines and recommendations on mitigating and preventing the presence of nitrosamines in human medicinal products. Based on the details in the published CHMP Article 5(3) opinion and the Q&A document on its implementation, EMA/409815/2020, the acceptable intake (Al) limit of nitroso cytisine impurity was determined.

When a new nitrosamine is identified, in this case N-nitroso cytisine, but there is insufficient substance-specific data to derive a substance-specific limit for lifetime exposure as recommended by the ICH M7(R1) guideline, the Threshold of Toxicological Concern (TTC) of 18 ng/day (derived from the Lhasa carcinogenic potency database) can be used as the default option, as stated in point 10 of the Q&A document's calculation of the limit.

To convert an above limit (ng) to a specification limit (ppm) for a specific medicinal product is calculated by dividing the respective above limit (ng) by the maximum daily dose (mg) of a given product as reflected in the SmPC. Because the cytisine reference product 1.5 mg film coated tablets (Tabex^{®}) are taken 6 times daily (every 2 hours) as per SmPC, the maximum daily dose (MDD) of 9 mg was used to calculate the Al limit for N-nitroso cytisine and, consequently, for the liquid aqueous composition of the present invention. As a result, the Al limit for N-nitroso cytisine in products containing cytisine is set at 2 parts per million (ppm) using the formula above.

The inventor(s) of the present invention unexpectedly found that adding at least one antioxidant from the group consisting of a sulfite, bisulfite, and metabisulfite compound to the formulation in the pH range of 5.0 to 7.0, preferably in the range of 5.2-6.7, more preferably 5.5-6.4, and most preferably 5.8-6.1 limits the N-nitroso cytisine impurity below the acceptable intake (Al) level of 2 ppm, under accelerated conditions (40°C/75% Relative humidity (RH), for a prolonged period of time, thus improving the shelf-life of the liquid aqueous composition.

In one embodiment, at least one antioxidant from the group consisting of a sulfite, bisulfite, and metabisulfite compound acts as an inhibitor in the prevention of nitrosamine formation from secondary amines by reacting with the source of the nitrosation reaction, such as nitrites in excipients, which potentially form nitrosating agents under specific conditions and could react with vulnerable secondary amines to form N-Nitroso cytisine impurity.

The inventor(s) of the present invention also found that when using at least one antioxidant selected from the group consisting of a sulfite, bisulfite, and metabisulfite compound, the pH range of the liquid aqueous pharmaceutical composition of 5.0-7.0 is critical in maintaining the level of known impurities such as N-formylcytisine (FO), N-methyl cytisine (MO) at ≤0.5% each, and single unknown impurity at ≤0.20% and total impurities not exceeding 1.0% under accelerated storage conditions. A significant increase in chemical impurities above its acceptable limit was observed at pH values below 5.0 and above 7.0; additionally, above pH 7.0, a change in the physically appearance of the composition at elevated temperature was observed; specifically, the color of the composition turns yellow after 1 month of storage at 40°C/75% RH.

The inventor(s) of the present invention experimented with a number of different antioxidant and pH range combinations to stabilize the liquid aqueous composition, but it was surprisingly found that chemical stability, as well as the stability against oxidative degradation and/or nitrosation, are only observed when at least one antioxidant selected from the group consisting of a sulfite, a bisulfite, and a metabisulfite were added to the liquid aqueous composition at a pH range of 5.0 to 7.0, preferably in the range of 5.2-6.7, more preferably 5.5-6.4, and most preferably 5.8-6.1.

It is well known that as the fraction of the undissociated drug substance rises, so does its permeability through the oral mucosa. It was critical that cytisine not be absorbed into the body via the oral cavity. The high oral absorption of cytisine, which is present at alkaline pH in an undissociated form, is predicted to affect the bioequivalence of the liquid aqueous composition in comparison to cytisine in tablet form. The inventor(s) of the present invention found a way to circumvent this issue by developing a liquid aqueous composition that contains at least one antioxidant selected from the group consisting of a sulfite, bisulfite, and metabisulfite compound that is bioequivalent to cytisine tablets (Tabex^{®}) by maintaining the pH value of the liquid composition in the range of from 5.0 to 7.0, preferably in the range of 5.2-6.7, more preferably 5.5-6.4, and most preferably 5.8-6.1.

It is widely accepted in the industry that antimicrobial preservatives may be added to pharmaceutical preparations, particularly aqueous preparations, if the preparation itself does not have sufficient antimicrobial activity. This is done to stop microbial contamination from occurring in the product under normal storage and use conditions, especially for multidose containers, which could endanger the patient by infecting them and causing the preparation to spoil. To make sure that such activity has not been hampered by storage, the antimicrobial activity of the preparation in its final container is examined over the course of the shelf-life. A pharmaceutical preparation must demonstrate, both during development and throughout its shelf-life, that its antimicrobial activity helps protect against adverse effects that could arise from microbial contamination or proliferation during storage and use of the preparation, or, if necessary, with the addition of a suitable preservative or preservatives.

The inventor(s) of the present invention found that by adding an antioxidant selected from the group consisting of a sulfite, bisulfite, and metabisulfite compound in the pH range of 5.0 to 7.0, preferably in the range of 5.2-6.7, more preferably 5.5-6.4, and most preferably 5.8-6.1, the liquid aqueous pharmaceutical of the present invention can be preserved against microbiological growth, eliminating the need to add any additional preservative such as but not limited to paraben M, paraben P, or sodium benzoate as used in prior art documents such as RU2593585C1 and WO2021115977A1.

Surprisingly, the inventor(s) of the present invention found that the shelf-life of liquid aqueous composition is increased by stabilizing the liquid composition against nitrosation and/or oxidative degradation, being chemically stable, preventing microbiological growth, and being bioequivalent to the reference tablet (Tabex^{®}), and all of these effects are achieved only by adding at least one antioxidant selected from the group consisting of a sulfite, bisulfite, and metabisulfite compound to the liquid aqueous composition in the pH range of 5.0 to 7.0, preferably in the range of 5.2-6.7, more preferably 5.5-6.4, and most preferably 5.8-6.1.

In one embodiment of the present invention, the liquid aqueous pharmaceutical composition further comprises one or more pharmaceutically acceptable excipient(s).

The pharmaceutical excipients used for preparing the liquid aqueous pharmaceutical composition of the present invention are known in the art can be chosen by a skilled person depending on their function. Reference can be made in this context to the Handbook of Pharmaceutical Excipients, ed. by Paul J Sheskey et al. 9th edition, Pharmaceutical Press (2020).

According to one embodiment of the liquid aqueous pharmaceutical composition of the present invention, one or more pharmaceutically acceptable excipient(s) are selected from a group consisting of a co-solvent, a sweetener, and a flavoring agent.

The liquid aqueous pharmaceutical composition of the present invention may comprise at least one co-solvent to aid in the dissolution of additives in the composition.

In one embodiment of the liquid aqueous pharmaceutical composition of the present invention, the co-solvent is selected from a group consisting of propylene glycol, polyethylene glycol, glycerin, and mixture thereof.

In one embodiment of the liquid aqueous pharmaceutical composition of the present invention, the co-solvent is selected from a group consisting of propylene glycol, polyethylene glycol, glycerin, and mixture thereof and wherein the co-solvent is present in an amount of from 5% to 50% by weight, based on the total weight of the pharmaceutical composition.

To enhance the flavor of the liquid aqueous pharmaceutical composition, the liquid aqueous pharmaceutical composition of the present invention may also include at least one sweetener and/or at least one flavoring agent.

In one embodiment of the liquid aqueous pharmaceutical composition of the present invention, the sweetener is selected from erythritol, fructose, dextrose, saccharin, sorbitol, xylitol, mannitol, maltose, maltitol, maltitol solution, liquid glucose, inulin, isomalt, saccharin sodium, sodium cyclamate, sucralose, sucrose, acesulfame potassium, or aspartame.

In the most preferred variant of the above embodiment, the sweetener is selected from, xylitol, mannitol, acesulfame K, aspartame, erythritol, maltitol, or sucrose and wherein the sweetener is present in an amount of from 1% to 20% by weight, based on the total weight of the pharmaceutical composition.

In one embodiment of the liquid aqueous pharmaceutical composition of the present invention, the flavoring agent at least one flavor can be selected from the group comprising mint flavor, tropical flavor, banana flavor, cherry flavor or orange flavor.

In one embodiment of the liquid aqueous pharmaceutical composition of the present invention, wherein the flavoring agent is selected from the group consisting of mint flavor, tropical flavor, banana flavor, cherry flavor, and orange flavor and wherein the flavoring agent is present in an amount of from 0.01% to 5% by weight, based on the total weight of the pharmaceutical composition.

In one embodiment, the liquid aqueous pharmaceutical composition according to the present invention comprises:

| **Ingredient** | **Amount (% by weight, based on the total weight of the pharmaceutical composition)** |
|---|---|
| Cytisine | 0.1-10% |
| Sweetener | 1-20% |
| pH regulating agent | 0.05-10% |
| Co-solvent | 5-50% |
| Antioxidant | 0.01-1% |
| Flavoring agent | 0.01-5% |
| Water, purified | up to 100% |

In a preferred variant of the above embodiment, the liquid aqueous pharmaceutical composition according to the present invention comprises:

| **Ingredient** | **Amount (% by weight, based on the total weight of the pharmaceutical composition)** |
|---|---|
| Cytisine | 0.1-10% |
| Xylitol | 1-20% |
| Sodium dihydrogen phosphate dihydrate | 0,05-10% |
| Glycerol | 5-50% |
| Propylene glycol | |
| Sodium metabisulfite | 0.01-1% |
| Liquid mint aroma | 0.01-5% |
| Water, purified | up to 100% |

One embodiment of the present invention is the method for preparing a stabilized liquid aqueous composition comprising cytisine or its pharmaceutically acceptable salt thereof, comprising.
i) providing aqueous cytisine solution,
ii) adding at least one antioxidant selected from the group consisting of a sulfite, bisulfite, and metabisulfite compound,
iii) optionally, adding further pharmaceutically acceptable excipient(s),
iv) adjusting the pH thereof to a value in the range of 5.0-7.0 by adding at least one inorganic pH regulating agent, and
v) finally, water is added to prepare the final volume.
wherein at least one antioxidant selected from the group consisting of a sulfite, bisulfite, and metabisulfite compound is present in an amount of from 0.01 % to 2.0 % by weight, based on the total weight of the pharmaceutical composition.

In one embodiment, when making an aqueous cytisine solution, 90% of the total amount of water used for the composition is used for cytisine dissolution before adding at least one antioxidant selected from the group consisting of a sulfite, bisulfite, and metabisulfite compound, optional pharmaceutically acceptable excipient(s), and at least one inorganic pH regulating agent to adjust the pH in the range of 5.0-7.0. The remaining water added in the final step of preparing the composition to the final volume has no impact on the pH range of 5.0-7.0.

In one embodiment, the liquid aqueous pharmaceutical composition of the present invention is suitable for administration as a mist, a spray, or an aerosol.

In one embodiment, the liquid aqueous pharmaceutical composition of the present invention can be transferred to a bottle fitted with an atomizer capable of dispensing 1.5 mg of cytisine per actuation.

One embodiment of the present invention provides a liquid aqueous pharmaceutical composition comprising cytisine, and at least one antioxidant selected from the group consisting of a sulfite, bisulfite, and metabisulfite compound, wherein at least one antioxidant is present in an amount of from 0.01 % to 2.0 % by weight, based on the total weight of the pharmaceutical composition, and wherein the pH of the said liquid aqueous composition is in the range of 5.0-7.0, preferably in the range of 5.2-6.7, more preferably 5.5-6.4, and most preferably 5.8-6.1, for oral use in the treatment of tobacco smoking addiction and other forms of nicotine addiction.

One embodiment of the present invention is the use of at least one antioxidant selected from the group consisting of a sulfite, bisulfite, and metabisulfite compound for the stabilization of a liquid aqueous pharmaceutical composition comprising cytisine, wherein at least one antioxidant is present in an amount ranging from 0.01% to 2.0% by weight, based on the total weight of the pharmaceutical composition, and wherein the pH of said liquid aqueous composition is in the range of 5.0-7.0, preferably in the range of 5.2-6.7, more preferably 5.5-6.4, and most preferably 5.8-6.1.

The present invention includes the following embodiments:
1. A liquid aqueous pharmaceutical composition comprising cytisine or a pharmaceutically acceptable salt thereof, at least one antioxidant selected from the group consisting of a sulfite, bisulfite, and metabisulfite compound, wherein the antioxidant is present in an amount of from 0.01 % to 2.0 % by weight, based on the total weight of the pharmaceutical composition and wherein the pH of the said liquid aqueous composition is in the range of 5.0-7.0.
2. The liquid aqueous pharmaceutical composition according to embodiment 1, wherein the antioxidant is selected from the group consisting of a sulfite, bisulfite, and metabisulfite compound is present in an amount of from 0.01 % to 1.0 % by weight, based on the total weight of the pharmaceutical composition.
3. The liquid aqueous pharmaceutical composition according to embodiments 1 or 2, wherein the antioxidant is selected form sodium sulfite, potassium sulfite, sodium bisulfite, potassium bisulfite, sodium metabisulfite, and potassium metabisulfite.
4. The liquid aqueous pharmaceutical composition according to embodiment 3, wherein the antioxidant is sodium sulfite or sodium metabisulfite.
5. The liquid aqueous pharmaceutical composition according to any one of embodiments 1-4, wherein the pH of the of liquid aqueous pharmaceutical composition is 5.2-6.7, more preferably 5.5-6.4, and most preferably 5.8-6.1.
6. The liquid aqueous pharmaceutical composition according to any one of embodiments 1-5, wherein cytisine or a pharmaceutically acceptable salt thereof is present in an amount which corresponds to from 0.1 % to 10 % by weight of cytisine free base, based on the total weight of the pharmaceutical composition.
7. A liquid aqueous pharmaceutical composition according to any one of embodiments 1-6, wherein the liquid aqueous pharmaceutical composition further comprises one or more pharmaceutically acceptable excipient(s).
8. The liquid aqueous pharmaceutical composition according to any one of embodiments 1-7, having the following composition:

| **Ingredient** | **Amount (% by weight, based on the total weight of the pharmaceutical composition)** |
|---|---|
| Cytisine | 0.1-10% |
| Sweetener | 1-20% |
| pH regulating agent | 0.05-10% |
| Co-solvent | 5-50% |
| Sulfiting agent | 0.01-1% |
| Flavoring agent | 0.01-5% |
| Water, purified | up to 100% |

9. The liquid aqueous pharmaceutical composition according to any one of embodiments 1-8, having the following composition:

| **Ingredient** | **Amount (% by weight, based on the total weight of the pharmaceutical composition)** |
|---|---|
| Cytisine | 0.1-10% |
| Xylitol | 1-20% |
| Sodium dihydrogen phosphate dihydrate | 0,05-10% |
| Glycerol | 5-50% |
| Propylene glycol | |
| Sodium metabisulfite | 0.01-1% |
| Liquid mint aroma | 0.01-5% |
| Water, purified | up to 100% |

10. The liquid aqueous pharmaceutical composition according to any one of embodiments 1-9 for use in the treatment of tobacco smoking addiction and other forms of nicotine addiction.
11. A liquid aqueous pharmaceutical composition for use according to embodiment 10, wherein the liquid composition is for administration into the oral cavity of a subject by mist, a spray or aerosol.
12. Use of at least one antioxidant selected from the group consisting of a sulfite, bisulfite, and metabisulfite compound for the stabilization of a liquid aqueous pharmaceutical composition comprising cytisine, wherein the antioxidant is present in an amount of from 0.01 % to 2.0 % by weight, based on the total weight of the pharmaceutical composition, and wherein the pH of said liquid aqueous composition is in the range of 5.0-7.0.

### Experimental part

### Measuring instruments, conditions, and protocols:

### Method for measuring the pH:

The pH is measured according to the European Pharmacopeia 2.2.3 at room temperature (20-25°C) and atmospheric pressure using a potentiometric pH meter apparatus (such as 913 pH Meter Lab, LL-Unitrode easy Clean 1m) calibrated using 2.00, 4.01 and 7.00 buffer solutions.

### Detection of chemical purity:

Chemical purity analysis was carried out using High Performance Liquid Chromatograph equipped with PDA detector, sample cooling system and column thermostat.

| | |
|---|---|
| HPLC column | Zorbax XDB C-18, 150 mm x 4,6 mm, 5 µm, |
| Flow | 0,8 ml/min, |
| Column temperature | 25°C, |
| Detection | 305 nm, |
| Injection volume | 20 µl, |
| Analysis time | 43 min, |
| Solvent | phosphate buffer pH 2.5: methanol (90:10, v/v) |
| Mobile Phases | A: phosphate buffer pH 2.5 + 0.1 % Sodium 1-heptanesulfonate |
| | B: methanol |

### Detection of N-Nitroso cytisine impurity:

Analysis for detection of acceptable intake (Al) level of N-nitroso cytisine was carried out using a liquid chromatograph with MS detector e.g., Shimadzu Nexera X2 LC MS 8040 or equivalent equipped with Phenomenex Luna Omega 1.6µm 2.1x100 mm column.

The following examples describe the present invention in detail but are not to be construed to limit the present invention.

### Example 1: Preparation of liquid composition of cytisine using sodium metabisulphite as antioxidant at pH 6.1.

The quantitative composition of this formulation is shown in the table below. Cytisine was dissolved in 90% of the total amount of water used for the composition, along with sodium metabisulfite, followed by the addition of propylene glycol, xylitol, and mint flavor, and the pH of the formulation was adjusted to 6.1 by adding sodium dihydrogen phosphate dihydrate, before adding the remaining water to the desired volume.

The finished pharmaceutical composition is transferred to an atomizer-equipped HDPE bottle.

| Ingredients | Amount | |
|---|---|---|
| | mg | [%] |
| Cytisine | 1.50 | 0.7 |
| Xylitol | 21.35 | 10.0 |
| Sodium dihydrogen phosphate dihydrate | 6.40 | 3.0 |
| Sodium metabisulfite | 1.70 | 0.8 |
| Glycerol | 44.83 | 21.0 |
| Propylene glycol | 44.83 | 21.0 |
| Liquid mint aroma | 0.214 | 0.1 |
| Purified water | 93.0 | 43.4 |

### Example 2: Preparation of liquid composition of cytisine using sodium metabisulphite as antioxidant at pH 5.8.

The quantitative composition of this formulation is shown in the table below. Cytisine was dissolved in 90% of the total amount of water used for the composition, along with sodium metabisulfite, followed by the addition of propylene glycol, xylitol, and mint flavor, and the pH of the formulation was adjusted to 5.8 by adding sodium dihydrogen phosphate dihydrate, before adding the remaining water to the desired volume.

The finished pharmaceutical composition is transferred to an atomizer-equipped HDPE bottle.

| Ingredients | Amount | |
|---|---|---|
| | mg | [%] |
| Cytisine | 1.50 | 0.7 |
| Xylitol | 21.35 | 10.0 |
| Sodium dihydrogen phosphate dihydrate | 8.53 | 4.0 |
| Sodium metabisulfite | 2.13 | 1.0 |
| Glycerol | 44.83 | 21.0 |
| Propylene glycol | 44.83 | 21.0 |
| Liquid mint aroma | 0.214 | 0.1 |
| Purified water | 90.44 | 42.2 |

### Example 3: Preparation of liquid composition of cytisine using sodium sulfite as antioxidant at pH 6.1.

The quantitative composition of this formulation is shown in the table below. Cytisine was dissolved in 90% of the total amount of water used for the composition, along with sodium sulfite, followed by the addition of propylene glycol, xylitol, and mint flavor, and the pH of the formulation was adjusted to 6.1 by adding sodium dihydrogen phosphate dihydrate, before adding the remaining water to the desired volume.

The finished pharmaceutical composition is transferred to an atomizer-equipped HDPE bottle.

| Ingredients | Amount | |
|---|---|---|
| | mg | [%] |
| Cytisine | 1.50 | 0.7 |
| Xylitol | 21.35 | 10.0 |
| Sodium dihydrogen phosphate dihydrate | 6.40 | 3.0 |
| Sodium sulfite | 0.21 | 0.1 |
| Glycerol | 44.83 | 21.0 |
| Propylene glycol | 44.83 | 21.0 |
| Liquid mint aroma | 0.214 | 0.1 |
| Purified water | 94.5 | 44.1 |

### Example 4: Preparation of liquid composition of cytisine using sodium bisulfite as antioxidant at pH 6.1.

The quantitative composition of this formulation is shown in the table below. Cytisine was dissolved in 90% of the total amount of water used for the composition, along with sodium bisulfite, followed by the addition of propylene glycol, xylitol, and mint flavor, and the pH of the formulation was adjusted to 6.1 by adding sodium dihydrogen phosphate dihydrate, before adding the remaining water to the desired volume.

The finished pharmaceutical composition is transferred to an atomizer-equipped HDPE bottle.

| Ingredients | Amount | |
|---|---|---|
| | mg | [%] |
| Cytisine | 1.50 | 0.7 |
| Xylitol | 21.35 | 10.0 |
| Sodium dihydrogen phosphate dihydrate | 6.40 | 3.0 |
| Sodium bisulfite | 0.21 | 0.1 |
| Glycerol | 44.83 | 21.0 |
| Propylene glycol | 44.83 | 21.0 |
| Liquid mint aroma | 0.214 | 0.1 |
| Purified water | 94.5 | 44.1 |

### Comparative example 1: Preparation of liquid composition of cytisine using maleic acid as antioxidant at pH 6.1.

The quantitative composition of this formulation is shown in the table below. Cytisine was dissolved in 90% of the total amount of water used for the composition, along with maleic acid, followed by the addition of propylene glycol, xylitol, and mint flavor, and the pH of the formulation was adjusted to 6.1 by adding sodium dihydrogen phosphate dihydrate, before adding the remaining water to the desired volume.

| Ingredients | Amount | |
|---|---|---|
| | mg | [%] |
| Cytisine | 1.50 | 0.7 |
| Xylitol | 21.35 | 10.0 |
| Sodium dihydrogen phosphate dihydrate | 6.40 | 3.0 |
| Maleic acid | 0.04 | 0.02 |
| Glycerol | 44.83 | 21.0 |
| Propylene glycol | 44.83 | 21.0 |
| Liquid mint aroma | 0.21 | 0.1 |
| Purified water | 94.67 | 44.18 |

### Comparative example 2: Preparation of liquid composition of cytisine using sodium thiosulfate as antioxidant at pH 6.1.

The quantitative composition of this formulation is shown in the table below. Cytisine was dissolved in 90% of the total amount of water used for the composition, along with sodium thiosulfate, followed by the addition of propylene glycol, xylitol, and mint flavor, and the pH of the formulation was adjusted to 6.1 by adding sodium dihydrogen phosphate dihydrate, before adding the remaining water to the desired volume.

| Ingredients | Amount | |
|---|---|---|
| | mg | [%] |
| Cytisine | 1.50 | 0.7 |
| Xylitol | 21.35 | 10.0 |
| Sodium dihydrogen phosphate dihydrate | 6.40 | 3.0 |
| Sodium thiosulfate | 0.11 | 0.1 |
| Glycerol | 44.83 | 21.0 |
| Propylene glycol | 44.83 | 21.0 |
| Liquid mint aroma | 0.21 | 0.1 |
| Purified water | 94.5 | 44.1 |

### Comparative example 3: Preparation of liquid composition of cytisine using sodium ascorbate as antioxidant at pH 6.1.

The quantitative composition of this formulation is shown in the table below. Cytisine was dissolved in 90% of the total amount of water used for the composition, along with sodium ascorbate, followed by the addition of propylene glycol, xylitol, and mint flavor, and the pH of the formulation was adjusted to 6.1 by adding sodium dihydrogen phosphate dihydrate, before adding the remaining water to the desired volume.

| Ingredients | Amount | |
|---|---|---|
| | mg | [%] |
| Cytisine | 1.50 | 0.7 |
| Xylitol | 21.35 | 10.0 |
| Sodium dihydrogen phosphate dihydrate | 6.40 | 3.0 |
| Sodium ascorbate | 0.21 | 0.1 |
| Glycerol | 44.83 | 21.0 |
| Propylene glycol | 44.83 | 21.0 |
| Liquid mint aroma | 0.21 | 0.1 |
| Purified water | 94.5 | 44.1 |

### Comparative example 4: Preparation of liquid composition of cytisine using butylated hydroxytoluene (BHT) as antioxidant at pH 6.1.

The quantitative composition of this formulation is shown in the table below. Cytisine was dissolved in 90% of the total amount of water used for the composition, followed by the addition of BHT, propylene glycol, xylitol, and mint flavor, and the pH of the formulation was adjusted to 6.1 by adding sodium dihydrogen phosphate dihydrate, before adding the remaining water to the desired volume.

It is observed that BHT in the composition remains insoluble and present as a suspension.

| Ingredients | Amount | |
|---|---|---|
| | mg | [%] |
| Cytisine | 1.50 | 0.70 |
| Xylitol | 21.35 | 10.0 |
| Sodium dihydrogen phosphate dihydrate | 6.40 | 3.0 |
| BHT | 0.02 | 0.01 |
| Glycerol | 44.83 | 21.0 |
| Propylene glycol | 44.83 | 21.0 |
| Liquid mint aroma | 0.21 | 0.1 |
| Purified water | 94.69 | 44.19 |

### Comparative example 5: Preparation of liquid composition of cytisine without antioxidant at pH 6.1.

The quantitative composition of this formulation is shown in the table below. Cytisine was dissolved in 90% of the total amount of water used for the composition, followed by the addition of propylene glycol, xylitol, and mint flavor, and the pH of the formulation was adjusted to 6.1 by adding sodium dihydrogen phosphate dihydrate, before adding the remaining water to the desired volume.

| Ingredients | Amount | |
|---|---|---|
| | mg | [%] |
| Cytisine | 1.50 | 0.70 |
| Xylitol | 21.35 | 10.0 |
| Sodium dihydrogen phosphate dihydrate | 6.40 | 3.0 |
| Glycerol | 44.83 | 21.0 |
| Propylene glycol | 44.83 | 21.0 |
| Liquid mint aroma | 0.21 | 0.1 |
| Purified water | 94.36 | 44.20 |

### Comparative example 6: Preparation of liquid composition of cytisine at pH 4.5

The quantitative composition of this formulation is shown in the table below. Cytisine was dissolved in 90% of the total amount of water used for the composition, followed by the addition of propylene glycol, xylitol, and mint flavor, and the pH of the formulation was adjusted to 4.5 by adding citrate buffer, optionally stabilized with HCl or NaOH solution to pH 4.5, before adding the remaining water to the desired volume.

| Ingredients | Amount |
|---|---|
| | [%] |
| Cytisine | 0.70 |
| Xylitol | 10.0 |
| Sodium metabisulfite | 0.8 |
| Citric acid mono hydrate | 2.0 |
| Sodium citrate dihydrate | 1.25 |
| Glycerol | 21.0 |
| Propylene glycol | 21.0 |
| Liquid mint aroma | 0.1 |
| HCl/NaOH for pH regulation | |
| Purified water | 43.15 |

### Comparative example 7: Preparation of liquid composition of cytisine at pH 8.7

The quantitative composition of this formulation is shown in the table below. Cytisine was dissolved in 90% of the total amount of water used for the composition, followed by the addition of propylene glycol, xylitol, and mint flavor, and the pH of the formulation was adjusted to 8.7 by adding phosphate buffer, before adding the remaining water to the desired volume.

| Ingredients | Amount |
|---|---|
| | [%] |
| Cytisine | 0.70 |
| Xylitol | 10.0 |
| Sodium metabisulfite | 0.08 |
| Sodium dihydrogen phosphate dihydrate | 0.1 |
| Glycerol | 21.0 |
| Propylene glycol | 21.0 |
| Liquid mint aroma | 0.07 |
| Purified water | 47.05 |

### Comparative example 8: Liquid composition of cytisine according to example 2 of RU2593585

The liquid cytisine composition is prepared as described in Example 2 of RU2593585, and its quantitative composition is shown in the table below:

| RU2593585 (example 2) | |
|---|---|
| Ingredients | Amount |
| | mg |
| Cytisine | 10.0 |
| NaH2PO4 dihydrate | 250.0 |
| Na2HPO4 anhydrous | 200.0 |
| Paraben M | 300.0 |
| Paraben P | 7.0 |
| EDTA | 10.0 |
| Citric acid | 10.0 |
| Sodium chloride | 8400.0 |
| Polysorbate | 500.0 |
| Water | Add to obtain 1 L of solution |
| pH | 7.7 |

### Comparative example 9: Liquid composition of cytisine according to composition 13 of international patent application WO2021115977A1.

The liquid cytisine composition is prepared as described in composition 13 of international patent application WO2021115977A1, and its quantitative composition is shown in the table below:

| WO2021115977 (composition 13) | |
|---|---|
| Ingredients | Amount |
| | [%] |
| Cytisine | 0.70 |
| Xylitol | 7.00 |
| Sodium hyaluronate | 0.10 |
| Mint flavor | 0.15 |
| NaH2PO4 dihydrate | 3.00 |
| Paraben M | 0.10 |
| Glycerol anhydrous | 21.00 |
| Polyethylene glycol | 15.00 |
| Water | ad 100% |
| pH | 6.1 |

### Comparative study of the chemical stability of liquid pharmaceutical compositions using various antioxidants.

The chemical stability of the present invention's liquid aqueous composition containing sodium metabisulfite, and sodium sulfite as antioxidants, as described in example 1, and 3, at pH 6.1, was compared to the liquid composition containing different antioxidants at pH 6.1, as described in comparative examples 1 and 3 was studied under accelerated storage conditions (40°C/75% RH). All samples were stored in a climate chamber, and samples were taken at time 0, 1, and 2 month intervals and analyzed by HPLC.

The stability study's results suggest that, in contrast to the compositions of examples 1 and 3, which contain sodium metabisulfite and sodium sulfite, respectively, as antioxidants, a significant increase in chemical impurities above its acceptable limit was observed at pH 6.1 under accelerated storage conditions using a different antioxidant.

| Impurities | Example 1 | Example 3 | Comparative example 1 | Comparative example 3 |
|---|---|---|---|---|
| Time (0) | | | | |
| FO | ND | NO | 0.01% | 0.03% |
| ME | NO | ND | ND | 0.02% |
| Total impurities* | 0.03% | 0.03% | 0.20% | 0.86% |

| 40°C/75% RH, 1 month | | | | |
|---|---|---|---|---|
| FO | 0.01% | 0.01% | 0.03% | 0.10% |
| ME | ND | NO | 0.02% | 0.03% |
| Total impurities* | 0.04% | 0.05% | 1.35% | 3.29% |

| 40°C/75% RH, 2 months | | | | |
|---|---|---|---|---|
| FO | 0.01% | 0.01% | 0.08% | 0.16% |
| ME | ND | NO | 0.03% | 0.07% |
| Total impurities* | 0.09% | 0.10% | 2.42% | 4.56% |
| ND - Not detected. | | | | |
| *Total impurities include known impurities, FO, ME as well as all unknown impurities | | | | |

### Comparative study of the chemical stability of liquid pharmaceutical compositions at different pH levels.

The chemical stability of the present invention's liquid aqueous composition containing sodium metabisulfite and sodium sulfite as antioxidants, as described in examples 1 and 3, at pH 6.1, was compared to the liquid composition containing sodium metabisulfite as antioxidants at pH 4.5 and 8.7, as described in comparative examples 6 and 7, and composition from example 2 of RU2593585 at pH 7.7 (comparative example 8), was studied under accelerated storage conditions (40°C/75% RH). All samples were stored in a climate chamber, and samples were taken at time 0, 1, and 2 months intervals and analyzed by HPLC. All chemical impurity content values are expressed in percentage (%).

The stability study's results suggest that, under accelerated storage conditions, chemical impurities significantly increased above the acceptable limit at pH values below 5.0 and above 7.0. However, the compositions of Examples 1 and 3 are stable and meet the requirements for pharmaceutical drug products containing cystine (FO impurity ≤0.5%, ME impurity ≤0.5%, max single unknown impurity ≤0.2%, total impurities ≤1.0%).

The accelerated study of the composition from Example 2 of RU2593585 (comparative example 8) lasted 6 months, and the total impurities were greater than 1.0%, indicating that the pharmaceutical drug product with cystine did not meet the specifications.

| Impurities | Example 1 | Example 3 | Comparative example 6 (pH 4.5) | Comparative example 7 (pH 8.7) | Comparative example 8 (pH 7.7) |
|---|---|---|---|---|---|
| Time (0) | | | | | |
| FO | NO | ND | ND | 0.83% | 0.05% |
| ME | NO | ND | ND | ND | ND |
| Total impurities* | 0.03% | 0.03% | 0.17% | 2.16% | 0.20% |

| 40°C/75% RH, 1 month | | | | | |
|---|---|---|---|---|---|
| FO | 0.01% | 0.01% | <0.03% | 1.59% | 0.08% |
| ME | NO | ND | ND | 0.09% | 0.03% |
| Total impurities* | 0.04% | 0.05% | 2.43 | 5.19% | 0.25% |

| 40°C/75% RH, 2 months | | | | | |
|---|---|---|---|---|---|
| FO | 0.01% | 0.01% | 0.03% | 2.28% | 0.10% |
| ME | NO | ND | ND | 0.16% | 0.04% |
| Total impurities* | 0.09% | 0.10% | 3.52% | 6.67% | 0.46% |
| ND - Not detected. | | | | | |
| *Total impurities include known impurities, FO, ME as well as all unknown impurities | | | | | |

### Comparative study of N-nitroso cystine content in liquid pharmaceutical compositions with various antioxidants, without antioxidants, and prior art compositions.

N-nitroso cytisine content in liquid pharmaceutical compositions of the present invention's containing sodium metabisulfite, sodium sulfite and sodium bisulfite as antioxidants, as described in example 1, 3 and 4, at pH 6.1, was compared to the liquid composition containing different antioxidants, as described in comparative examples 1, 2, 3, 4, and comparative example 5 (without antioxidant) and prior art compositions as described in comparative examples 8 and 9 was studied under normal storage (25°C/75% RH) and accelerated storage conditions (40°C/75% RH). All samples were stored in a climate chamber, and samples were taken at 1 and 6 month intervals and analyzed by LCMS, with the content of N-nitroso cytisine impurity expressed in parts per million (ppm).

The stability study's results suggest that, in contrast to the compositions of examples 1, 3, and 4, which contain sodium metabisulfite, sodium sulfite and sodium bisulfite, as antioxidants, respectively, a significant increase in the content of N-nitroso cytisine impurity was observed under accelerated storage conditions in comparative example 8 and above its acceptable intake (Al) limit of 2 ppm with liquid composition containing different antioxidants, as described in comparative examples 1, 2, 3, 4, and comparative example 5 (without antioxidant), and comparative example 9.

| Examples | N-Nitroso cytisine content [ppm] | | |
|---|---|---|---|
| | 25°C/60% | 40°C/75% | 40°C/75% |
| | RH, 1 month | RH, 1 month | RH, 6 months |
| Example 1 (sodium metabisulfite) | <0.2 | <0.2 | <0.2 |
| Example 3 (sodium sulfite) | 0.28 | 0.31 | 0.35 |
| Example 4 (sodium bisulfite) | 0.20 | 0.22 | 0.26 |
| Comparative example 1 (maleic acid) | 1.83 | 2.12 | NA |
| Comparative example 2 (sodium thiosulfate) | 1.79 | 4.59 | NA |
| Comparative example 3 (sodium ascorbate) | 0.38 | 0.42 | 2.12 |
| Comparative example 4 (BHT) | 3.04 | 3.15 | NA |
| Comparative example 5 (without antioxidant) | NA | 2.56 | 4.90 |
| Comparative example 8 (RU2593585) | 0.2 | 0.36 | 0.84 |
| Comparative example 9 (WO2021115977A1) | 1.29 | 1.77 | 3.86 |
| NA - Not analyzed | | | |

### Comparative bioavailability study

The comparative bioavailability of the following cytisine dosage form was tested.
- Cytisine, 1.5 mg oral solution of Example 1, administered in a single dose under fasting conditions (A).
- Cytisine, 1.5 mg oral solution of Example 1, administered in a single dose under fasting conditions and drinking water after the dose (B).
- 1.5 mg film coated tablet Tabex^{®}, given in a single dose with water under fasting conditions.

A randomized, open-label, single dose, three period, pilot, cross-over bioavailability study was carried out on 24 healthy volunteers under fasting condition.

Randomization scheme was generated by SAS^{®}/Plan module as shown in the table below and blood samples were collected for the period of 24 hours.

| The number of treatments in this trial = 3 Seq No | Period1 | Period2 | Period3 |
|---|---|---|---|
| 1 | A | C | B |
| 2 | B | A | C |
| 3 | C | B | A |
| 4 | B | c | A |
| 5 | C | A | B |
| 6 | A | B | C |
| A = Test product - Cytisine, oral solution, 1.5 mg given in a single dose without water under fasting conditions. | | | |
| B = Test product - Cytisine, oral solution, 1.5 mg given in a single dose with water under fasting conditions. | | | |
| C = Reference product - Tabex^{®} 1.5 mg film coated tablet given in a single dose with water under fasting conditions. | | | |

The comparative bioavailability data (AUC, Cₘₐₓ and Tₘₐₓ) obtained for the cytisine oral solutions (treatment A and B) vs Tabex^{®} (treatment C) are shown in the table below.

| Pharmacokinetic Parameter | Comparison | Point estimate | | Coefficient of variation (%) | | |
|---|---|---|---|---|---|---|
| | | Arithmetic mean 1 | Arithmetic mean 2 | Ratio (%) | Lower 90% Confidence Limit | Upper 90% Confidence Limit |
| AUC0-t(h.pg/mL) | A vs C | 85683.43 | 89605.87 | 95.62 | 93.12 | 98.19 |
| | B vs C | 84935.02 | 89605.87 | 94.79 | 92.32 | 97.32 |
| | A vs B | 85683.43 | 84935.02 | 100.9 | 98.25 | 103.6 |
| Cmax(pg/mL) | A vs C | 13114.87 | 14842.78 | 88.36 | 80.64 | 96.82 |
| | B vs C | 14487.19 | 14842.78 | 97.60 | 89.10 | 106.9 |
| | A vs B | 13114.87 | 14487.19 | 90.53 | 82.62 | 99.19 |

Surprisingly, it was found that the liquid aqueous composition of the present invention, is bioequivalent to the reference product Tabex^{®}.

### Test for efficacy of antimicrobial preservation

The compositions of examples 1 and 3 were tested for antimicrobial preservation efficacy. The compositions were tested with a prescribed inoculum of microorganisms (as listed in the table below), the inoculated preparation was stored at a prescribed temperature, and the samples, typically 1 ml or 1 g, were withdrawn from the container at zero hour and at appropriate intervals (14 days and 28 days) and the number of viable microorganisms were determined by plate count.

The acceptance criteria for antimicrobial activity evaluation are specified in *Ph. Eur. 5.1.3* in terms of the Iog10 reduction in the number of viable microorganisms against the value obtained for the inoculum. The preservative properties of the preparation are adequate if, during the test, there is a significant decrease or no increase, as appropriate, in the number of microorganisms in the inoculated preparation after the times and at the temperatures specified.

As shown in the table below, the compositions of examples 1 and 3 show no increase in the number of microorganisms in the inoculated preparation after 14 and 28 days, respectively, and thus satisfy the requirement specified in Ph. Eur. 5.1.3.

| | Example 1 | | Example 3 | |
|---|---|---|---|---|
| | 14 days | 28 days | 14 days | 28 days |
| Concentration of sodium metabisulfite | 0.8 % | | - | |
| Concentration of sodium sulfite | - | | 0.1 % | |
| Staphylococcus aureus | 5.8 | 5.8 | 5.8 | 5.8 |
| Pseudomonas aeruginosa | 6.2 | 6.2 | 5.8 | 5.8 |
| Escherichia coli | 6.2 | 6.2 | 6.0 | 6.0 |
| Zygosaccharomyces bisporus | 6.3 | 6.3 | 5.7 | 5.7 |
| Candida albicans | 5.7 | 5.7 | 5.8 | 5.8 |
| Aspergillus brasiliensis | 6.3 | 6.3 | 6.1 | 6.1 |

## Claims

1. A liquid aqueous pharmaceutical composition comprising cytisine or a pharmaceutically acceptable salt thereof, at least one antioxidant selected from the group consisting of a sulfite, bisulfite, and metabisulfite compound, wherein the antioxidant is present in an amount of from 0.01% to 2.0% by weight, based on the total weight of the pharmaceutical composition and wherein the pH of the said liquid aqueous composition is in the range of 5.0-7.0.

2. The liquid aqueous pharmaceutical composition according to claim 1, wherein the antioxidant selected from the group consisting of a sulfite, bisulfite, and metabisulfite compound is present in an amount of from 0.01% to 1.0% by weight, based on the total weight of the pharmaceutical composition.

3. The liquid aqueous pharmaceutical composition according to claim 1 or 2, wherein the antioxidant is selected form sodium sulfite, potassium sulfite, sodium bisulfite, potassium bisulfite, sodium metabisulfite, and potassium metabisulfite.

4. The liquid aqueous pharmaceutical composition according to claim 3, wherein the antioxidant is sodium sulfite or sodium metabisulfite.

5. The liquid aqueous pharmaceutical composition according to any one of claims 1-4, wherein the pH of the liquid aqueous pharmaceutical composition is 5.2-6.7, more preferably 5.5-6.4, and most preferably 5.8-6.1.

6. The liquid aqueous pharmaceutical composition according to any one of claims 1-5, wherein cytisine or a pharmaceutically acceptable salt thereof is present in an amount which corresponds to from 0.1 % to 10 % by weight of cytisine free base, based on the total weight of the pharmaceutical composition.

7. A liquid aqueous pharmaceutical composition according to any one of claims 1-6, wherein the liquid aqueous pharmaceutical composition further comprises one or more pharmaceutically acceptable excipient(s).

8. The liquid aqueous pharmaceutical composition according to any one of claims 1-7 for use in the treatment of tobacco smoking addiction and other forms of nicotine addiction.

9. A liquid aqueous pharmaceutical composition for use according to claim 8, wherein the liquid composition is for administration into the oral cavity of a subject by mist, a spray or aerosol.

10. Use of at least one antioxidant selected from the group consisting of a sulfite, bisulfite, and metabisulfite compound for the stabilization of a liquid aqueous pharmaceutical composition comprising cytisine, wherein the antioxidant is present in an amount of from 0.01% to 2.0% by weight, based on the total weight of the pharmaceutical composition, and wherein the pH of said liquid aqueous composition is in the range of 5.0-7.0.
